# EUROPEAN PATENT APPLICATION

(11) **EP 4 119 682 A1**
(43) Date of publication of application: **18.01.2023**
(21) Application number: 21305977.7
(22) Date of filing: 13.07.2021
(51) Int. Cl.: C12Q 1/70, C12Q 1/6806

(54) **A METHOD FOR DETERMINING THE NATURE OF DNA IMPURITIES INSIDE VIRAL PARTICLES**

(71) Applicant: GENETHON, 91000 Evry (FR)
(72) Inventor: ALCINDOR, Jérémie, 91200 ATHIS-MONS (FR); BEARD, Nicolas, 91410 DOURDAN (FR); DORANGE, Fabien, 28630 NOGENT-LE-PHAYE (FR)
(74) Representative: Cabinet Laurent & Charras

(57) **Abstract**

The present invention concerns a method for determining the nature of DNA impurities inside recombinant viral particles. The method comprises isolating the viral capsids in a closed environment, disrupting the viral capsids, specifically eliminating the DNA released from the viral capsids depending on whether it is single-stranded or double-stranded, and detecting and/or quantifying the remaining target DNA. The selective elimination of of nucleic acids can be by nuclease digestion.The present invention concerns a method for determining the nature of DNA impurities inside recombinant viral particles. The method comprises isolating the viral capsids in a closed environment, disrupting the viral capsids, specifically eliminating the DNA released from the viral capsids depending on whether it is single-stranded or double-stranded, and detecting and/or quantifying the remaining target DNA. The selective elimination of of nucleic acids can be by nuclease digestion.

## Description

The present invention relates to the production of recombinant viral particles useful in gene therapy. It provides a method which allows determining the nature (single-stranded (ss) or double-stranded (ds)) of the nucleic acid molecules, and notably contaminants, contained in said particles and then assessing their safety.

### BACKGROUND OF THE INVENTION

Gene therapy (also called human gene transfer) is a medical field which focuses on the utilization of the therapeutic delivery of nucleic acids into a patient's cells as a drug to treat diseases. It is thought to be able to cure many genetic disorders or treat them over time.

The concept of gene therapy is to fix a genetic problem at its source. If, for instance, in an (usually recessively) inherited disease a mutation in a certain gene results in the production of a dysfunctional protein, gene therapy could be used to deliver a copy of this gene that does not contain the deleterious mutation (named transgene), and thereby produces a functional protein. This strategy is referred to as gene replacement therapy. Alternatively, antisense therapy using antisense oligonucleotides (AON) e.g. allowing exon skipping, or silencing therapy (RNA interference) using small inhibitory RNA (siRNA) can be used.

For example, documents WO2015/197869 and WO2006/021724 illustrate the treatment of Duchenne muscular dystrophy (DMD), based on gene replacement therapy and antisense therapy, respectively.

Most of these approaches utilize adeno-associated viruses (AAVs) and lentiviruses for performing nucleic acid insertions, *in vivo* and *ex vivo,* respectively. In more than 75% of gene therapy clinical trials, a viral vector is the vehicle.

Adeno-associated virus (AAV) is the most common and powerful vector, derived from adeno-associated human parvovirus. This small virus has attracted the attention of the gene therapy field because of its low risk (not pathogenic for human and no integrative genome), the remarkable ability of recombinant AAV (rAAV) vectors to transduce with high efficiency dividing and non-dividing cells in a large variety of tissues, long-term transgene expression after injection and specific tissue tropism.

Adeno-associated virus (AAV) is a small non-enveloped DNA virus composed by an icosahedral capsid that contains a 4.7 kb linear single-stranded genome. AAV genome codes for non-structural proteins (Rep78, 68, 52 and 40), capsid proteins (VP1, VP2, VP3) and the assembly-activating protein (AAP). At the extremities, inverse tandem repeats (ITR) are important for encapsidation of the viral genome. AAV is a defective virus requiring the presence of a helper virus for an active infection. Twelve distinct serotypes and more than 100 natural variants have been identified.

In practice, the nucleic acid sequence of interest, usually placed under the control of regulatory sequences allowing its expression, replaces the viral genome between the ITR sequences.

Recombinant viral particles can be obtained by methods known in the art, especially by co-transfection of 293 HEK cells. The generation of rAAV vectors entails encapsidation of a therapeutic gene of interest utilizing the replication (rep) and capsid (cap) genes. rAAV production requires delivery of the proviral (vector DNA) construct and the rep and cap genes specific to the serotype of interest.

Adenovirus (Ad) and HSV have been shown to be necessary for rAAV replication. In regard to the Ad helper virus, the *E1a, E1b, E2a, E4orf6, E1B55K* and *VA RNA* genes were determined to supply the helper functions necessary for rAAV production by supporting AAV protein synthesis (Meier et al., 2020, Viruses, 12(6): 662). Infection of Ad into producer cells to generate rAAV was effective in producing rAAV, but a consequence was that it also produced Ad particles. A significant improvement in the evolution of rAAV production was the introduction of the triple plasmid transfection method. This method used an AAV serotype-specific rep and cap plasmid as well as the vector DNA plasmid, but eliminated the use of Ad infection by supplying the essential Ad genes on a third plasmid (pXX6). Supplying the Ad helper genes on the pXX6 plasmid eliminated Ad production in the transfected cells yielding only rAAV vector.

AAV replicate by a strand displacement mechanism, using the ITR as a primer to initiate second-strand synthesis resulting in a new covalently closed dsDNA molecule. To resolve the duplex structure, the terminal resolution site (trs) within the ITR sequence is nicked by Rep78 or Rep68, and the remaining part is converted into dsDNA. AAV capsid assembly is supported by the viral assembly-activating protein (AAP), and its requirement ranges broadly across serotypes. Newly synthesized AAV DNA is packaged into preassembled capsids. This process requires the helicase activity of the small Rep proteins, Rep40 and Rep52, which function as a motor for loading the ssDNA genome into capsids. A large Rep protein, covalently attached to newly formed AAV genomes, might serve as a packaging signal as it was shown that p5-containing (and ITR-lacking) sequences, which are bound by Rep, can be packaged into AAV-capsids.

The so obtained viral particles can contain single-stranded (ss) or double-stranded (ds) acid nucleic molecules.

In order to avoid as many side effects as possible, these AAV vectors are used without their pathogenic and replicative parts. However, it is still very important to control and follow the manufacturing process to ensure that no unwanted elements (contaminants) are found in the product.

In practice and in relation to gene therapy, residual host cell DNA and plasmid DNA are product related contaminants of AAV vectors. These DNA are encapsidated in AAV vectors during the production steps. They contribute to safety issue as these impurities can induce an immune response and/or oncogenicity. The nature of these DNA has an impact on the risk issue. Indeed, a single-stranded DNA (ssDNA) should be less prone to DNA integration in transduced cells than double-stranded DNA (dsDNA). Determination of the nature of DNA contaminants should help to increase the safety of the final product.

The use of these gene therapy vectors with structural and technical complexity greater than all drugs available today is subject to regulation by health authorities. Numerous analytical tests are in place to ensure the quality and safety of these gene therapy vectors. Some of them are dedicated to the control and quantification of residual or contaminant DNA, especially DNA from plasmids and host cells.

The most used technology is qPCR ("quantitative Polymerase Chain Reaction") which allows quantifying and monitoring vector genome (VG) and residual DNA during all the process of production and purification. For example, the resDNAseq kit (Thermofisher) is used to quantify and monitor host cell residual DNA without target differentiation. Actually, to quantify the residual Host Cell DNA (e.g. AdE1a, AdE1b, 18S sequences....) and residual DNA backbone plasmid sequences including resistance and origin of replication, qPCR and digital PCR technologies are used. These technologies make it possible to quantify and monitor each target on each AAV batch at each step of the process.

However, they do not give any information on the DNA nature (i.e. ss or ds) of the targets, and notably the contaminants.

To solve this problem, protocols and kits (e.g. Qubits kits, Quantit oligreen and picogreen) have been developed using dyes which recognize the specific nature of DNA. However and in general, kits or methods based on the measurement of DNA with a fluorescent dye are not very specific: They do not allow a specific detection of one type of DNA in a mixture because the dyes are not entirely specific and cross-react in case of mixed ssDNA and dsDNA. The two types of DNA are then quantified in different proportions. Moreover and in presence of both types of target, they quantify the all DNA in a sample but cannot quantify one specific DNA target. In conclusion, they do not render possible the accurate identification and quantification of DNA of different nature.

Other techniques like southern-blot can help to determine the nature of DNA by changing the migration condition in a gel. Recently, the possibility to determine the nature of DNA by southern blotting, by comparing the DNA impurity profiles, was described (Hauck et al., 2009, Mol. Ther., 17(1): 144-52.). However, such a method has several disadvantages, especially because the exact nature of the DNA remains hypothetic and it is not very sensitive rendering the quantification not possible:
Southern blot is a DNA gel migration method. It is possible to migrate the DNA in denaturing/non-denaturing conditions that has an impact on the DNA shape. The advantage of southern blot is that it permits to target specific DNA sequences. This method cannot demonstrate the nature of a target as such, but the accumulation of data in denaturing and non-denaturing conditions allows formulating hypotheses about it.

Since the discontinuation of the use of radioactive probes to target DNA sequences and the use of less sensitive detection methods, less suppliers sell kits. Therefore, southern-blot experiments require performing hand-made protocols.

In addition, despite the use of a large amount of sample, the southern blot is not a very sensitive method: It is impossible to detect and quantify small quantities of specific residual DNA sequences.

More of concern, all these methods require the use of purified nucleic acid preparation and extraction procedure. In the case of AAV vectors, the extraction of both positive and negative single-stranded AAV genome, encapsidated separately, can trigger the hybridization of both strands and so finally change the nature of the DNA. In the same way, single-stranded DNA impurities such as plasmids and host cell DNA can become double-stranded by re-annealing with complementary sequences. For these reasons, determination of the AAV genome and DNA impurities nature using an extraction step is inappropriate as the nature of the encapsidated DNA may be modified.

Therefore, there is still a need to provide a method which allows determining the nature of a specific targeted DNA, without being impacted by a possible modification of its nature during determination.

### BRIEF SUMMARY OF THE INVENTION

As defined by the claims, the present invention relates to a method based on partitioning the viral particles in an individual environment, e.g. a droplet. This results in the partition of an individual nucleic acid, especially DNA molecule, in the droplet that can be further analyzed to establish its nature without the bias introduced by re-annealing of complementary DNA.

To the best knowledge of the inventors, this is the first report of a process which allows directly determining the nature of DNA without the risk of potential annealing of complementary sequences during the process.

### Definitions

Unless otherwise defined, all technical and scientific terms used therein have the same meaning as commonly understood by one of ordinary skill in the art. The terminology used in the description is for the purpose of describing particular embodiments only and is not intended to be limiting.

The articles "a" and "an" are used herein to refer to one or to more than one (*i.e.,* to at least one) of the grammatical object of the article. By way of example, "an element" means one element or more than one element.

"About" or "approximately" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 2, from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

A "nucleotide sequence encoding an amino acid sequence" includes all nucleotide sequences that are degenerate versions of each other and that encode the same amino acid sequence. The phrase nucleotide sequence that encodes a protein or a RNA or a cDNA may also include introns to the extent that the nucleotide sequence encoding the protein may in some version contain an intron(s).

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR and the like, and by synthetic means.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

"Identical" or "homologous" refers to the sequence identity or sequence similarity between two polypeptides or between two nucleic acid molecules. When a position in both of the two compared sequences is occupied by the same base or amino acid monomer subunit, e.g., if a position in each of two DNA molecules is occupied by adenine, then the molecules are homologous or identical at that position. The percent of homology/identity between two sequences is a function of the number of matching positions shared by the two sequences divided by the number of positions compared X 100. For example, if 6 of 10 of the positions in two sequences are matched then the two sequences are 60% identical. Generally, a comparison is made when two sequences are aligned to give maximum homology/identity.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus (AAV) vectors, retroviral vectors, and the like.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (*e.g.,* naked or contained in liposomes) and viruses (*e.g.,* lentiviruses, retroviruses, adenoviruses, and adeno-associated viruses) that incorporate the recombinant polynucleotide.

As used herein, the term "adeno-associated virus" or "AAV" has its general meaning in the art and refers to a Dependoparvovirus within the Parvoviridae genus of viruses. The term can refer to an AAV derived from a naturally occurring "wild-type" virus, or an AAV derived from a rAAV genome packaged into a capsid derived from capsid proteins encoded by a naturally occurring cap gene. The term "AAV" includes human AAV type 1 (AAV-1), AAV type 2 (AAV-2), AAV type 3 (AAV-3), AAV type 4 (AAV-4), AAV type 5 (AAV-5), AAV type 6 (AAV-6), AAV type 7 (AAV-7), AAV type 8 (AAV-8), AAV type 9 (AAV-9), AAV type rh74 (AAV-rh74) or any fusion between two or more of said AAV type, e.g. AAV type 9 fused with AAV type rh74 (AAV-9/rh74). Said AAV can also be fused with specific peptide, e.g. PI peptide (RGDLGLS). "Adeno-associated virus" or "AAV" also refers to avian AAV, bovine AAV, canine AAV, porcine AAV, equine AAV, primate AAV, non-primate AAV, and ovine AAV. "Primate AAV" refers to AAV that infect primates, "non-primate AAV" refers to AAV that infect non primate mammals, "bovine AAV" refers to AAV that infect bovine mammals, etc. The genomic sequences of various serotypes of AAV, as well as the sequences of the native inverted terminal repeats (ITRs), Rep proteins, and capsid subunits are known in the art. Such sequences may be found in the literature or in public databases such as GenBank.

As used herein, the term "cap gene" refers to the polynucleotide that encode capsid proteins that form, or contribute to the formation of, the capsid, or protein shell, of the virus. In the case of AAV2, the capsid protein may be VP1, VP2, or VP3. For other parvoviruses, the names and numbers of the capsid proteins can differ.

As used herein, the term "rep gene" refers to the nucleic acid sequences that encode the non-structural proteins (rep78, rep68, rep52 and rep40) required for the replication and production of virus.

As used herein, the term "packaging" refers to a series of intracellular events that result in the assembly and encapsidation of an AAV virus.

A "helper virus" for AAV refers to a virus that allows AAV (e.g. wild-type AAV) to be replicated and packaged by a mammalian cell. A variety of such helper viruses for AAV are known in the art, including adenoviruses, herpesviruses and poxviruses such as vaccinia. The adenoviruses encompass a number of different subgroups, although Adenovirus type 5 of subgroup C is most commonly used. Numerous adenoviruses of human, non-human mammalian and avian origin are known and available from depositories such as the ATCC. Viruses of the herpes family include, for example, herpes simplex viruses (HSV) and Epstein- Barr viruses (EBV), as well as cytomegaloviruses (CMV) and pseudorabies viruses (PRV); which are also available from depositories such as ATCC. "Helper virus function(s)" refers to function(s) encoded in a helper virus genome which allow AAV replication and packaging (in conjunction with other requirements for replication and packaging described herein). As described herein,"helper virus function" may be provided in a number of ways, including by providing helper virus or providing, for example, polynucleotide sequences encoding the requisite function(s) to a producer cell in trans. For example, a plasmid or other expression vector comprising nucleotide sequences encoding one or more adenoviral proteins is transfected into a producer cell along with nucleotidic sequences enconding for the AAV rep and cap genes and a transgene expression cassette flanked by two ITRs to produce a rAAV.

As used herein, the term "virus viral particle" is one that comprises a competently assembled viral capsid and is capable of delivering a polynucleotide component into a cell for which the viral species is tropic. The term does not necessarily imply any replication capacity of the virus. Assays for counting infectious viral particles are described elsewhere in this disclosure and in the art. Viral infectivity can be expressed as the ratio of infectious viral particles to total viral particles.

As used herein, the term "transgene" or "gene of interest" refers to a polynucleotide that is introduced into a cell and is capable of being transcribed into RNA and optionally, translated and/or expressed under appropriate conditions. In aspects, it confers a desired property to a cell into which it is introduced, or otherwise leads to a desired therapeutic outcome. In another aspect, it may be transcribed into a molecule that mediates RNA interference, such as miRNA, siRNA, or shRNA. In some embodiments, the gene of interest is a functional version of a gene or a fragment thereof. The functional version of said gene includes the wild-type gene, a variant gene such as variants belonging to the same family and others, or a truncated version, which preserves the functionality of the encoded protein at least partially. A functional version of a gene is useful for replacement or additive gene therapy to replace a gene, which is deficient or non-functional in a patient. In other embodiments, the gene of interest is a gene which inactivates a dominant allele causing an autosomal dominant genetic disease. A fragment of a gene is useful as recombination template for use in combination with a genome editing enzyme. Alternatively, the gene of interest may encode a protein of interest for a particular application, (for example an antibody or antibody fragment, a genome-editing enzyme) or a RNA. In some embodiments, the protein is a therapeutic protein including a therapeutic antibody or antibody fragment, or a genome-editing enzyme. In some embodiments, the RNA is a therapeutic RNA.

### DETAILED DESCRIPTION OF THE INVENTION

The present invention concerns a method for characterizing the nucleic acids (NA) contained in viral capsids. More precisely, the present method allows determining the nature of the nucleic acids (NA) contained in viral capsids, and notably of the contaminants.

According to a preferred embodiment, the nucleic acids (NA) which are characterized, i.e. which nature is determined by the method of the invention, are deoxyribonucleic acids (DNA).

In the frame of the present application, the expression "determining the nature of NA" or "determining the nature of DNA" advantageously means determining if the nucleic acid (NA) or desoxyribonucleic acid (DNA) molecules contained in the viral capsids are single-stranded (ss) or double-stranded (ds).

According to a specific embodiment, determining the nature of the nucleic acids contained in viral capsids comprises discriminating or distinguishing or differentiating ss and ds nucleic acids, advantageously ss and dsDNA. It is to be noted that in some cases, the nucleic acids can be self complementary (sc), i.e. form an intra-molecular double-stranded (ds)NA. In other words, the method according to the invention allows discriminating or distinguishing or differentiating ss nucleic acids on one hand, and ds or sc nucleic acids on another hand.

In the frame of the present application, the expression "viral capsids" has a common meaning, i.e. it designates the shell, advantageously the protein shell of the virus. In addition, "viral particles" (or virions) are usually defined as at least a nucleic acid molecule surrounded by a protective coat called a capsid, or a protein capsid containing genetic material. In the rest of the description and for simplification purposes, the viral capsids and the viral particles as used herein are interchangeable.

According to one embodiment, the viral capsid contains at least one acid nucleic molecule.

In the frame of the invention, the origin of said acid nucleic(s) can vary:
The viral capsid usually contains the viral genome (VG), which can be single-stranded (ss) DNA, double-stranded (ds) DNA, self-complementary (sc) DNA or RNA. In relation to gene therapy, the following viral vectors are commonly used:
- Adenoviruses (e.g. Ad5) which genome is dsDNA;
- AAV (e.g. AAV8, AAV9) which genome is usually ssDNA but which can also be dsDNA or self complementary (sc);
- Herpes simplex (e.g. HSV1, HSV) which genome is dsDNA;
- Retroviruses (e.g. MMSV, MSCV) which genome is ssRNA;
- Lentiviruses (e.g. HIV-1, HIV-2) which genome is ssRNA;
- Alphaviruses (e.g. SFV, SIN, VEE, M1) which genome is ssRNA;
- Flaviviruses (e.g. Kunjin, West Nile, Dengue virus) which genome is ssRNA;
- Rhabdoviruses (e.g. Rabies, VSV) which genome is ssRNA;
- Measles virus (e.g. MV-Edm) which genome is ssRNA;
- Newcastle disease virus which genome is ssRNA;
- Poxviruses (e.g. VV) which genome is dsDNA;
- Picornaviruses (e.g. Coxsackievirus) which genome is ssRNA.

According to a specific embodiment, the viral particle contains a recombinant viral genome, i.e. wherein a transgene or a gene of interest as defined above has been introduced.

In relation to gene therapy, the nature of the viral genome (especially DNA or RNA; ss, sc or ds) is usually known and depends on the virus used. Moreover, said viral genome is the nucleic acid molecule of interest, potentially encoding a therapeutic product.

Detection and quantification of the viral genome, especially in case of DNA genome, is usually performed using classical methods in the art, such as Polymerase Chain Reaction (PCR) or more recent versions thereof such as digital Polymerase Chain Reaction (dPCR). Primers and probes can target the transgene or specific sequences of the viral genome such as the ITR sequences for AAV vectors. The vector titers are usually expressed as viral genomes per mL (vg/mL).

As known in the art, the viral capsids can however contain other nucleic acids, especially deoxyribonucleic acids, e.g. inadvertently collected during the production process and packaged inside the capsids. Such nucleic acids have no therapeutic value or even worth can have negative effects, e.g. oncogenic and/or immunogenic activity. Therefore, said nucleic acids, which exclude the viral genome, are referred to as "impurities" or "contaminants" or "residual(s)".

As explained above and of particular concern are the "impurities" or "contaminants" which are in the form of dsDNA. Indeed, the safety of a gene therapy product is usually evaluated on said criterion.

Said "impurities" or "contaminants" encompass any exogenous nucleic acids, especially deoxyribonucleic acids and in particular dsDNA, originating from the tools used for the production of the viral particles including the cells, the virus, the plasmids, ...

Examples of such acid nucleic are:
- sequences originating from classical plasmids, e.g. conserved sequence (e.g. origin of replication of *Escherichia Coli*) and/or antibiotic (e.g. Kanamycin, Ampicillin) resistance;
- sequences originating from the cells and plasmids dedicated to the production of viral particles, e.g. the Ad helper gene (e.g. *E2*)*,* the *rep* gene or the *cap* gene;
- sequences originating from the cells used for the production of the viral particles, e.g. from the HEK293 cells used for the production of AAV vectors such as ribosomal sequence, Ad helper gene (*E1A*) or Large T Antigen sequence;
- sequences originating from the virus used for the production of the viral particles, e.g. from baculoviruses.

A list of possible primers and probes, to be used e.g. in PCR or dPCR in relation to AAV8 or AAV9 vectors harboring a GFP (Green Fluorescent Protein) protein and produced by co-transfection of 293 HEK cells, is shown in the examples.

According to the invention, said method is performed on isolated viral particles. Thus, the analysis of the content of the capsid(s) prevents or reduces artefacts due to hybridization between nucleic acids contained in different capsids.

First, the method according to the invention comprises isolating the capsid(s) from a sample in a closed environment.

According to a specific embodiment, the closed environment is a droplet, advantageously an oil droplet, as generated when performing droplet digital Polymerase Chain Reaction (ddPCR) as disclosed by Hindson et al. (2011, Anal. Chem. 83(22): 8604-10), also named dPCR.

In the frame of the invention, a sample is usually a liquid medium containing the viral capsids. It can be buffer, advantageously an aqueous buffer, in which the viral capsids are collected or resuspended e.g. for injection, or may result from their production.

According to a further specific embodiment, isolating an individual capsid in a closed environment comprises fractioning a sample containing the viral capsids using the water-oil emulsion droplet technology. This can be achieved using a ddPCR System, e.g. the Automated Droplet Generator sold by BIO-RAD. In practise, a sample is fractioned in thousands of droplets and PCR amplification of the template molecules occurs in each individual droplet.

Before generating the droplet, the sample is dilute in an appropriate manner in order to isolate the capsid(s) in the droplet. According to a preferred embodiment, only one viral particle is contained in each droplet. More generally, this step should allow getting one target sequence detectable in each droplet. In case the target DNA of interest is in a very low amount, its detection by the method according to the invention can require more than one viral particle (i.e. at least one target DNA sequence) in the droplet. Advantageously, this number is as low as possible, i.e. no more than 5, 4 or even 3, e.g. 2 or 3.

Further, the isolated capsid is disrupted or disassembled. In the frame of the invention, "disrupting" and disassembling" are used interchangeably, and mean that the physical integrity of the viral capsid is affected so that the nucleic acids contained therein, i.e. the viral genome and possible undesired nucleic acids, are released in the closed environment, advantageously the droplet. Because of the closed environment, the released nucleic acids cannot anneal with other nucleic acids not contained in said viral capsid or closed environment.

Any condition or agent which allows disruption or disassembly of the viral capsid can be used, as long as it is compatible with the closed environment, e.g. a droplet, advantageously an oil droplet. In an appropriate manner, said condition or agent specifically acts on the viral capsid, without affecting the other components present in the closed environment, especially the nucleic acids or any further agent required for the implementation of the method of the invention.

Said condition or agent is determined by the skilled person and depends on the nature and the properties of the viral capsid. In particular, any condition or agent able to degrade or denature the capsid proteins, e.g. VP1, VP2 and VP3 in case of an AAV, can be used.

Examples of conditions or agents are:
- Proteolytic enzymes including nonspecific proteases (proteinase K, cathepsine, ...) or more specific proteases (Factor Xa Protease, Enterokinase, Furin, trypsine, α-Lytic Protease, Endoproteinase GluC, Endoproteinase AspN, Endoproteinase LysC, TEV Protease...);
- Chaotropic agents such as urea or guanidinium chloride;
- Reducing agents (thiol, mercaptoethanol, Dithiothreitol (DTT)...);
- Detergents;
- Temperature;
- pH;
- Pressure;
- Stirring ...

In case the closed environment is a droplet, especially an oil droplet, said agent is preferably added before generating said droplets, i.e. in the sample of viral capsids.

According to a specific embodiment, capsid disruption occurs during a heating step or treatment at an adequate temperature, depending of the nature of the viral capsid. In the case of AAV8 and AAV9 vectors, a minimal temperature allowing the disassembly is at least 71.2°C for AAV8 (empty in PBS) and 77°C for AAV9 (in PBS) (Bennett et al., 2017, Mol Ther Methods Clin Dev, 24(6): 171-82). As an example and in such a case, a temperature of 80°C may be applied. The duration of the heating step is easily determined by the skilled person, e.g. 5 minutes for AAV8 or AAV9 vectors.

A further step of the method according to the invention is the specific degradation or elimination of a part of the deoxyribonucleic acids contained in the viral capsid, depending on the nature thereof.

As mentioned above, the nature of said nucleic acids can be single-stranded (ss) on one hand and double-stranded (ds) or self-complementary (sc) on another hand.

According to a specific embodiment, said step allows discriminating ss and ds/sc nucleic acids, advantageously ssDNA and ds/scDNA.

Any agent or condition which allows differential degradation or elimination of nucleic acids can be implemented in the method according to the invention, as long as it is compatible with the closed environment, e.g. a droplet, advantageously an oil droplet, and with the agent or condition used for capsid disassembly.

According to a specific embodiment, the degradation or elimination takes place by enzyme digestion.

According to one embodiment, the enzyme is a nuclease, i.e. an enzyme able to cleave the nucleic acids. Advantageously, the implemented nuclease is a deoxyribonuclease (DNAse). According to one embodiment, the DNAse specifically degrades DNA but not RNA.

Among these nucleases, some of them specifically digest single-stranded (ss) nucleic acids whereas others specifically digest double-stranded (ds) nucleic acids.

According to a preferred embodiment, the nuclease specifically degrades dsDNA. Examples of such nucleases are: Duplex-Specific Nuclease (Evrogen); dsDNAse (ArticZymes); dsDNAse (#EN0771; Thermofisher); Exonuclease III; Lambda Exonuclease; Exonuclease VIII, truncated; T7 Exonuclease and T7 Exonuclease I.

According to another embodiment, the nuclease specifically degrades ssDNA. Examples of such nucleases are: Exonuclease I; Exonuclease VII; Rec J Exonuclease; RecJf; Exonuclease T and Mung Bean Nuclease.

In case the closed environment is a droplet, especially an oil droplet, said enzyme is preferably added before generating said droplets, i.e. in the sample of viral capsids. In practise, the enzyme digestion starts when the viral capsid is disrupted, thereby releasing the nucleic acids in the closed environment.

The optimal temperature and incubation time depend on the DNAse implemented. The temperature and the duration of the enzyme digestion is easily determined by the skilled person or given by the provider's instructions.

According to a specific embodiment, degradation of double-stranded (ds)DNA occurs through incubation with the Duplex-Specific Nuclease (DSN; Evrogen). In particular, such an enzyme remains active at high temperatures, especially at the temperature applied for viral capsid disruption.

According to the provider, optimal digestion takes place at 65°C for 7 to 20 minutes. In order to ensure the complete degradation of ds/scDNA, a longer digestion, e.g. for 1 hour, can be performed, while slightly decreasing the temperature, e.g. at 60°C.

At the end of the digestion, the enzyme may be inactivated by any means of the art, e.g. by temperature denaturation or by physical removal of the enzyme or of the nucleic acids.

According to a specific embodiment, the elimination of the enzyme takes place in two steps :
- An inactivation at high temperature, e.g. at 95°C for 10 minutes;
- A disruption of the droplets with an extraction of the DNA. This second step allows eliminating non-inactivated enzyme.

As known by the skilled person, it is possible to control the correct inactivation of the enzyme: The elimination step(s) is performed on a sample containing no DNA (negative control) and DNA is added afterwards. If said DNA is degraded, it means that the inactivation of the DNAse was incomplete.

Following this treatment, the method comprises detecting and/or quantifying one or more target nucleic acid(s).

In the frame of the invention, "detecting" means assessing the presence or absence of said target sequence, whereas "quantifying" means measuring the level of said target sequence.

A target nucleic acid means a nucleic acid which can be specifically detected and/or quantified by means of specific tools, e.g. oligonucleotides specifically hybridizing with said nucleic acid.

According to a specific embodiment and in case of DNA, said detection or quantification is performed by PCR, advantageously by ddPCR, using adapted probes and primers.

Said step can require the recovery of the nucleic acids from the closed environment. In the case of oil droplets, a so-called droplet disruption protocol is disclosed in the examples.

Of particular interest, a target nucleic acid is not part of the viral genome, but is a suspected contaminant as defined above.

According to a preferred embodiment, the same method is performed without the degradation step, e.g. in the absence of the enzyme.

By comparing the treated (with enzyme; condition ① in the table below) and untreated sample (without enzyme; condition ② in the table below), it is possible to conclude on the nature of the nucleic acids contained in the viral capsids, especially of the target nucleic acid(s).

Merely as examples, the table below illustrates the conclusions which can be drawn based on the method of the invention in relation to ssDNA and dsDNA:

| Viral Genome (VG) | Potential Contaminant (C) | Enzyme | Nucleic acids after enzyme digestion | Conclusion based on target C (detection of C) |
|---|---|---|---|---|
| ssDNA e.g. AAV | ssDNA +dsDNA | dsDNAse ( 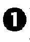 ) | VG + ssC | If 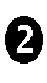 > 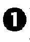 i.e. (ssC+dsC)>ssC: ds contaminants |
| | | No Enzyme ( 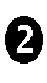 ) | VG + ssC + dsC | If 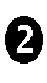 = 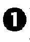 i.e. (ssC+dsC)=ssC: No ds contaminant |
| ssDNA e.g. AAV | ssDNA +dsDNA | ssDNAse ( 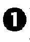 ) | dsC | If 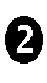 > 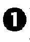 i.e. (ssC+dsC)>dsC: ss contaminants |
| | | No Enzyme ( 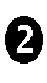 ) | VG + ssC + dsC | If 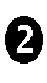 = 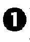 i.e. (ssC+dsC)= dsC: No ss contaminant |
| dsDNA e.g. Adenovirus or scDNA e.g. AAV | ssDNA +dsDNA | dsDNAse ( 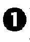 ) | ssC | If 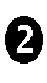 > 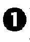 i.e. (ssC+dsC)>ssC: ds contaminants |
| | | No Enzyme ( 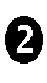 ) | VG+ ssC + dsC | If 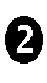 = 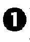 i.e. (ssC+dsC)=ssC: No ds contaminant |
| dsDNA e.g. Adenovirus or scDNA e.g. AAV | ssDNA +dsDNA | ssDNAse ( 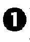 ) | VG + dsC | If 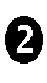 > 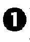 i.e. (ssC+dsC)>dsC: ss contaminants |
| | | No Enzyme ( 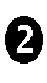 ) | VG+ ssC + dsC | If 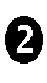 = 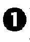 i.e. (ssC+dsC) = dsC: No ss contaminant |
| RNA e.g. Lentivirus | ssDNA +dsDNA | dsDNAse ( 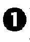 ) | VG + ssC | If 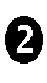 > 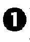 i.e. (ssC+dsC)>ssC: ds contaminants |
| | | No Enzyme ( 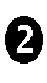 ) | VG + ssC + dsC | If 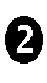 = 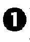 i.e. (ssC+dsC)=ssC: No ds contaminant |
| RNA e.g. Lentivirus | ssDNA +dsDNA | ssDNAse ( 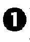 ) | VG + dsC | If 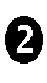 > 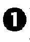 i.e. (ssC+dsC)>dsC: ss contaminants |
| | | No Enzyme ( 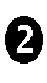 ) | VG+ ssC + dsC | If 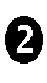 = 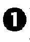 i.e. (ssC+dsC) = dsC: No ss contaminant |

According to a first aspect, the invention concerns a method for determining the nature of a target DNA contained in the viral capsids of a sample comprising:
- isolating the capsid(s) in a closed environment so as to have at least one target sequence;
- disrupting the viral capsid(s);
- specifically eliminating some of the DNA released from the viral capsid(s), depending on its nature;
- detecting and/or quantifying the remaining (i.e. not eliminated) target DNA.

According to another aspect, the invention concerns a method comprising:
- isolating capsid(s) and a specific nuclease in a droplet, advantageously in an oil droplet;
- disrupting the viral capsid by heat treatment, advantageously at 80°C;
- after nuclease treatment, detecting and/or quantifying a target DNA.

As explained above, performing the method of the invention without the step of eliminating the nucleic acids, especially DNA, e.g. in the absence of the nuclease, provides a negative control and allows concluding on the nature of the nucleic acids, especially DNA, contained in the viral capsids.

When performed using nucleases specifically eliminating single-stranded (ss) or double-stranded (ds) nucleic acids, the method of the invention allows estimating the presence of ds contaminants in the tested sample of viral capsids and then evaluating its safety for therapeutic and clinical use.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Oligonucleotide Synthesis" (Gait, 1984); "Culture of Animal Cells" (Freshney, 2010); "Methods in Enzymology" "Handbook of Experimental Immunology" (Weir, 1997); "Gene Transfer Vectors for Mammalian Cells" (Miller and Calos, 1987); "Short Protocols in Molecular Biology" (Ausubel, 2002); "Polymerase Chain Reaction: Principles, Applications and Troubleshooting" (Babar, 2011); "Current Protocols in Immunology" (Coligan, 2002). These techniques are applicable to the production of the polynucleotides and polypeptides of the invention, and, as such, may be considered in making and practicing the invention. Particularly useful techniques for particular embodiments will be discussed in the sections that follow.

The disclosures of each and every patent, patent application, and publication cited herein are hereby incorporated herein by reference in their entirety.

Without further description, it is believed that one of ordinary skill in the art can, using the preceding description and the following illustrative examples, make and utilize the compounds of the present invention and practice the claimed methods.

### EXPERIMENTAL EXAMPLES

The invention is further described in detail by reference to the following experimental examples and the attached figures. These examples are provided for purposes of illustration only, and are not intended to be limiting.

In particular, the invention is illustrated in relation to AAV8 and AAV9 viral particles harboring a GFP (Green Fluorescent Protein) transgene and obtained by co-transfection of 293 HEK cells.

### FIGURES:

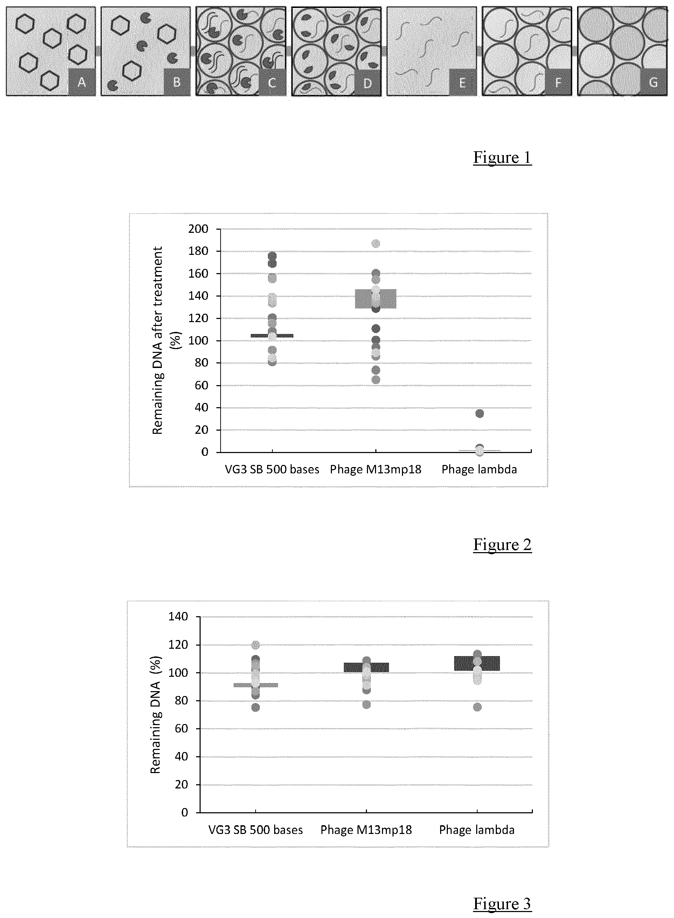

Figure 1: Scheme of a complete protocol of DNA nature characterization according to the invention
   (A) The AAV sample is dilute to have one capsid per droplet.
   (B) Duplex-specific nuclease (DSN) is added to the sample prior to droplet formation.
   (C) In the droplets, AAV capsids are disassembled by temperature, DSN treatment can start.
   (D) All double-stranded DNA are removed by DSN. DSN is inactivated.
   (E) Single-stranded DNA is recovered.
   (F) A droplet digital PCR is performed (G) to quantify sequence(s) of interest. A comparison between the treated and untreated sample by DSN allows concluding on DNA nature inside the AAV capsid.
Figure 2: Efficacy of DSN to differentiate ssDNA and dsDNA. The DNA from single-stranded 500 bases synthetic sequence (VG3), ssDNA from phage M13mp18 and dsDNA from phage lambda were treated and quantify by the complete protocol (n=48: 16 samples in triplicate).
Figure 3: Control of DSN inactivation. The DNA from single-stranded 500 bases sequence (VG3), ssDNA from phage M13mp18 and dsDNA from phage lambda were added to the negative controls containing only DSN that have followed the entire protocol.

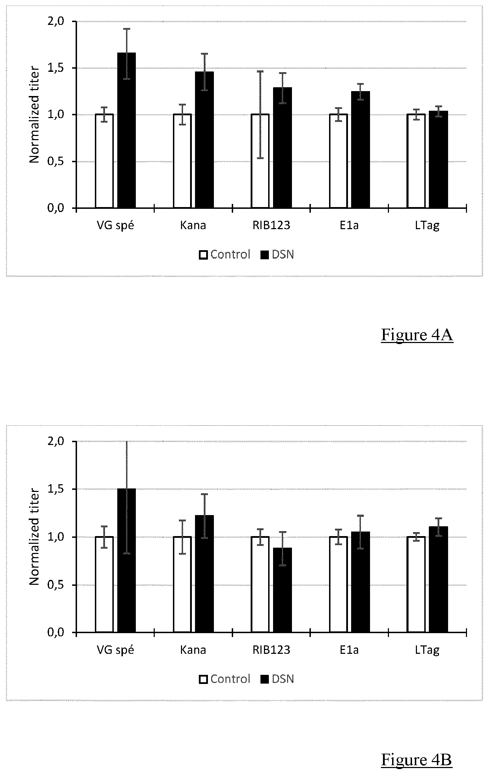

Figure 4: Determination of the DNA impurities nature inside AAV9-GFP vectors (batch 19DB187: A) and AAV8-GFP vectors (batch 19DB198: B). Five DNA sequences were analyzed, comprising VG spé (GFP), Kana (KanaR), RIB123 (ribosomal sequence), E1a, LTag (large T antigen). For each, the titer of untreated condition was normalized at 1, and the treated condition was adjusted in consequence. n = 6 samples.

### MATERIALS AND METHODS:

### AAV generation and purification

Vector generation was performed by helper virus-free transfection of HEK293 cells using three plasmids with modifications. The AAV8-GFP and AAV9-GFP vectors, batches 19DB198 and 19DB187 respectively, were generated using a vector plasmid containing a 6,537 bp backbone exceeding the 4 700 nt packaging limit of AAV. AAV8-GFP vector was generated using vector plasmid backbone of 7346 bp and AAV9-GFP vector was generated using vector plasmid backbone of 7340 bp. These two GFP vectors were generated using an helper plasmid of 18735 bp (Ayuso, E. et al. (2010) Current Gene Therapy, 10(6), 423-436). They express the Green-Fluorescent Protein (GFP). The purification starts with a clarification with an Express 0.5/0.2 filter, followed by cation-exchange chromatography using Poros 50HS (GE Healthcare, Piscataway, NJ) combined to a filtration step with an Express 0.5/0.2 filter. This step allows removing the totality of the residual DNA outside of the capsid. A last sterilizing filtration is performed on batch before use. Two AAV vectors (AAV8 and AAV9) were used in order to compare the nature of encapsidated DNA between these 2 serotypes.

### AAV Capsid isolation - Droplet generation

This step was performed using a device dedicated to ddPCR (droplet digital Polymerase Chain Reaction) as provided by Biorad (Automated Droplet Generator).

The titer of vector genome, Kana, RIB 123, E1a and LTag was previously determined by qPCR with the same primer/probe as those used for ddPCR quantification (see below). The dilution factor of each target was estimated by a comparison between the titer of each target (number copy per µl) and the average number of droplet formed by well.

As recommended by the manufacturer, it was chosen to work at 0.2 copy/droplet to be sure to statistically have 1 copy or less by droplet.

Sample was diluted in ringer lactate pluronic at appropriate dilution related to the target sequence.

Each well contains 10 µl of diluted sample, 5 µl of control DNA (composed by a mix of VG3 (IDT technologies), M13mp18 phage DNA (New England Biolabs), Lambda phage DNA (New England Biolabs) and 50µg/ml ETSSB (see below ***Nuclease treatment Duplex-Specific Nuclease)),*** 22 µl of ddPCR^{™} Buffer Control for Probes (Biorad) , 4.4 µl of DSN master mix (home made), 2µl of DSN (or 2µl of DSN buffer in case of untreated condition), 0,6 µl of water.

The mix is placed in the Automated Droplet Generator (Biorad) and 2 droplets generation are completed.

### Particle disassembly

A temperature of 80°C applied during 5 minutes was chosen to perform the disassembly for serotypes 8 and 9.

### Nuclease treatment Duplex-Specific Nuclease

The DSN (Duplex-Specific Nuclease; Evrogen) treatment was performed at 60°C during one hour. One unit (in 22µl of reaction mixture) of DSN was used to treat each sample. It is added in the reaction mixture as explained above (see ***AAV Capsid isolation* - *Droplet***

### generation).

DSN is inactivated by increasing temperature to 95°C during 10 minutes. DSN is further eliminated during the DNA extraction (see below ***DNA extraction* - *Droplet disruption*)*.*** An Extreme Thermostable ssDNA Binding Protein (ETSSB) (New England Biolabs) is added before DSN treatment in order to avoid formation of secondary structures and non-specific hybridization. This protein has a role in protection and stabilization of ssDNA.

### Control of DSN activity

DNA from phage lambda, M13mp18 or VG3 (synthetic ssDNA of 500 bases), which are dsDNA (phage lambda; positive control) and ssDNA (M13mp18 and VG3; negative control) respectively, was added to the sample in order to control the DSN treatment (see ***AAV Capsid isolation* - *Droplet generation*)*.***

### DNA extraction - Droplet disruption

DNA treated by DSN was recovered by a protocol of droplet disruption. The oil phase at the bottom of the tube was firstly discarded. A volume of elution buffer was added to the sample. A volume of chloroform was added, followed by a centrifugation at 16,000g during 10 minutes. The supernatant containing the DNA of interest was recovered.

### Control of DSN inactivation

After the DSN treatment and DNA extraction, a part of the negative control (containing only DSN) is used to perform a control of DSN inactivation. A mix of DNA from phage lambda, M13mp18 and VG3 was added to the negative control. A degradation of dsDNA spike in negative control indicates that DSN was not correctly inactivated. This control is perform to avoid false positive results due to an unwanted degradation of dsDNA formed by the hybridization of targeted DNA with a primer during PCR.

### Quantification of VG and residual DNA impurities by digital droplet PCR

Digital droplet PCR (ddPCR) was performed for AAV-8 and AAV-9 GFP VG (viral genome) quantification, using primers and probes specific for the GFP sequence as follows:
forward primer: 5'-AGTCCGCCCTGAGCAAAGA-3' (SEQ ID NO: 1)
reverse primer : 5'-GCGGTCACGAACTCCAGC-3' (SEQ ID NO: 2)
probe : 5'-CAACGAGAAGCGCGATCACATGGTC-3' (SEQ ID NO: 3).

Other transgenes were similarly quantified by ddPCR using primers and probes: Residual plasmid DNA was quantified by ddPCR using primers and probes specific for:
- the kanamycin resistance gene (KanaR) common to all plasmids used for rAAV vector generation:
   forward primer: 5'-GGGCGCCCGGTTCTTTTTGTC-3' (SEQ ID NO: 4)
   reverse primer : 5'-GCCAGTCCCTTCCCGCTTCAGTG-3' (SEQ ID NO: 5)
   probe : 5'- FAM-CCGACCTGTCCGGTGCCCTG-QSY-3' (SEQ ID NO: 6)
- the Adenovirus early region 1A (E1a) common for all serotypes and found on the helper plasmid used for rAAV vector generation:
   forward primer: 5'-ATGCCAAACCTTGTACCGGA-3' (SEQ ID NO: 7)
   reverse primer : 5'-CCCTCTTCATCCTCGTCGTC-3' (SEQ ID NO: 8)
   probe : 5'- FAM-ATCGATCTTACCTGCCACGAGGCTGG-QSY-3' (SEQ ID NO: 9).

Host cell DNA was quantified by ddPCR using primers and probes specific for:
- the ribosomal sequence found in HEK293 cells (RIB123):
   forward primer: 5'-GCAATTATTCCCCATGAACG-3' (SEQ ID NO: 10)
   reverse primer : 5'-GGCCTCACTAAACCATCCAA-3' (SEQ ID NO: 11)
   probe : 5'- FAM-AAGTCCCTGCCCTTTGTACACACCG-QSY-3' (SEQ ID NO: 12)
- large T antigen (LTag):
   forward primer: 5'-TTTAGATTGGCTAAGAAACAGTGATGA-3' (SEQ ID NO: 13)
   reverse primer : 5'-TTCCATGTTCTTCTCCCCACC-3' (SEQ ID NO: 14)
   probe : 5'- FAM-TGATGAAGACAGCCAGGAAAATGCTGATAAA-QSY-3' (SEQ ID NO: 15).

Single-stranded (ss) and double-stranded (ds) control DNA were also quantified by ddPCR using primers and probes specific for phage lambda, M13mp18 DNA and VG3:
- phage lambda (ds) :
   forward primer: 5'-ACAGCCCCTCGTTTATTATTTATCTC-3' (SEQ ID NO: 16)
   reverse primer : 5'-CCCGGCCTTTCTGTTATCC-3' (SEQ ID NO: 17)
   probe : 5'- FAM-TCAGCCAGCCGCTGTGCTTTCA-QSY-3' (SEQ ID NO: 18)
- phage M13mp18 (ss):
   forward primer: 5'-CCAGTTCTTTTGGGTATTCCGTTA-3' (SEQ ID NO: 19)
   reverse primer : 5'-GAAAAGTAAGCAGATAGCCGAACAA-3' (SEQ ID NO: 20)
   probe : 5'- VIC-GAAAAGTAAGCAGATAGCCGAACAA-QSY-3' (SEQ ID NO: 21).
- VG3 (ss):
   forward primer: 5'-TTTGACACTTCTTACAAACCCGTATC-3' (SEQ ID NO: 22)
   reverse primer : 5'-GAACTGTGGCAAGGAACAGGAT-3' (SEQ ID NO: 23)
   probe : 5'-VIC-CATTGTATACTGATTCTCG-QSY-3' (SEQ ID NO: 24).

### Determination of the nature of targeted DNA

A comparison between the corresponding treated and untreated samples is done. The titer obtained with the untreated sample is associated to the ratio 1. Based thereon, a ratio is calculated for the treated sample.

### RESULTS:

### AAV Capsid isolation in droplets (Figure 1A)

AAV capsids contain the vector genome (vg) and some residual DNA of plasmidic and cellular origin (contaminants). At the end of the production and purification process, all the DNA outside of the particles is removed via elimination/purification steps.

In ddPCR, drop-phase involves subdividing a reaction mixture into thousands of nanoliter-sized aqueous droplets in a water-oil emulsion. The content of a droplet can be predicted statistically by the Poisson law as reported by Brisco MJ et al. (1992 BioTechniques 13(3):444-9). In a known manner, there is a correlation between the sample concentration and the number of target in a droplet. For each DNA sequence analyzed, a dilution factor was calculated to have one or less target by droplet.

### Capsid disassembly inside droplet (Figure 1C)

An increase of temperature can induce DNA release from AAV capsids. In fact, the increase of temperature triggers the loss of secondary structures of VP1, VP2 and VP3 proteins. This was proved by the Differential Scanning Fluorimetry method (Bennett et al., 2017, Mol Ther Methods Clin Dev., 6: 171-182). Each serotype of AAV has a specific temperature of disassembly.

The serotypes AAV8 and AAV9, used in the study, have a disassembly temperature at 71.2°C and 77°C, respectively. In order to simplify the protocol, it was chosen to disassembly all the samples at 80°C which is sufficient to disassembly said capsid serotypes.

The protocol for determining the nature of the DNA in individual environment consists of isolating each capsid in a droplet in order to carry out the DNAse treatment. DNA is released from each capsid by heating the sample at 80°C for 5 minutes.

### DSN inside droplet and treatment (Figure 1B and 1D)

In order to differentiate ssDNA from dsDNA, DSN (duplex specific nuclease; Evrogen), an enzyme from Kamchatka crab that cleaves dsDNA compared to ssDNA and RNA, was used because of its specificity for dsDNA elimination: its degradation of dsDNA forms oligonucleotides avoiding false positive results. The chosen DSN is very sensitive to small dsDNA sequences smaller than 14 nucleotides and is resistant to high temperature until 90°C, thereby resisting to the step of DNA release and capsid disassembly.

Quantity of DSN per droplet was determined for efficient double-stranded DNA degradation.

The SSB protein was used to avoid ssDNA degradation by avoiding secondary structure formation. This eliminates errors related to non-specific degradation of ssDNA.

The complete protocol was performed on a mixture containing ssDNAs contaminants (synthetic VG3 and from phage M13mp18) and dsDNA contaminants from phage lambda **(****Figure 2****).** It reveals that DSN eliminates more than 90% of dsDNA whereas it shows no degradation on ssDNA with recovery of ssDNAs from 50 to 200 %. The difference of degradation between single and double-stranded DNA is significant.

### DSN inactivation

In order to avoid activity of residual DSN after enzyme treatment, an inactivation of the DSN was performed before the amplification step.

A control condition was added to the protocol in order to validate the DNAse inactivation before the amplification step. A negative control containing DSN that have followed the entire protocol is used. After DSN inactivation and DNA extraction step, the ssDNA VG3, ssDNA from phage M13mp18 and dsDNA from phage lambda was added. Complete inactivation of the DSN should lead to consistent recovery of the dsDNA from phage lambda.

**Figure 3** shows no degradation of the dsDNA from phage lambda which confirms the inactivation of DSN.

### Droplet disruption: Extraction of treated DNA (Figure 1E)

Following the disassembly of the capsids, DNAse can degrade all of the double-stranded DNA in the droplet. The ssDNA is then recovered from the droplets and then each target sequence representing residual DNA is quantified.

During this last step, it is possible that a part of the single-stranded DNA will hybridize with a homologous sequence from another droplet. The result will be processed as the presence or absence of a target. The presence of a target at the time of quantification means that it has not been degraded by DNAse, this target is then considered to be single stranded in the capsid.

### Analysis of the nature of DNA (Figure 1F)

The protocol was tested on an AAV9-GFP batch (19DB187) and an AAV8-GFP batch (19DB198). The absence of degradation of the viral genome (VG; ssDNA) was confirmed.

Then, the residual DNA impurities knew to be detected in AAV preparations, were evaluated:
- plasmidic target: kanamycin resistance gene (common of all plasmid involved in the production process));
- host-cell DNA targets: 18S ribosomal DNA (RIB 123), E1A and SV40 largeT antigen (LTag) DNAs.

**Figure 4**

## Claims

1. A method for determining the nature of a target deoxyribonucleic acid (DNA) contained in the viral capsids of a sample comprising:
- isolating the capsid(s) in a closed environment so as to have at least one target DNA sequence;
- disrupting the viral capsid(s);
- specifically eliminating the DNA released from the viral capsid(s), depending on its nature;
- detecting and/or quantifying the remaining target DNA.

2. The method according to claim 1, wherein the viral capsid is isolated in a droplet, advantageously an oil droplet generated by the water-oil emulsion droplet technology.

3. The method according to claim 1 or 2, wherein the viral capsid is disrupted by a heating treatment, advantageously at a temperature of 80°C for the viral capsid of an AAV8 or AAV9.

4. The method according to any of claims 1 to 3, wherein the DNA are specifically eliminated by nuclease digestion.

5. The method according to claim 4, wherein the nuclease specifically digests double-stranded (ds) DNA.

6. The method according to claim 4, wherein the nuclease specifically digests single-stranded (ss) DNA.

7. The method according to any of claims 4 to 6, wherein the nuclease is added to the sample before isolating the capsid(s), advantageously before generating the droplets.

8. The method according to any of the preceding claims, wherein the target DNA is detected and/or quantified by Polymerase Chain Reaction (PCR), advantageously by droplet digital PCR (ddPCR).

9. The method according to any of the preceding claims, wherein the target DNA is a contaminant DNA, advantageously originating from a plasmid or a host cell used to produce the viral capsids.

10. The method according to any of the preceding claims, wherein the viral capsids are from an adeno-associated virus (AAV).

11. The method according to any of the preceding claims, wherein the method is further performed without eliminating the DNA, advantageously in the absence of the nuclease.

12. Use of the method according to any of claims 1 to 11 to assess the quality of a viral gene therapy product.
